# EUROPEAN PATENT APPLICATION

(11) **EP 1 346 978 A1**
(43) Date of publication of application: **24.09.2003**
(21) Application number: 02252046.4
(22) Date of filing: 21.03.2002
(51) Int. Cl.: C07C 213/10, C07C 217/74

(54) **Process for preparing tramadol hydrochloride and/or tramadol monohydrate**

(71) Applicant: Jubilant Organosys Limited, Noida 201 301, Uttar Pradesh (IN)
(72) Inventor: Ansari, Shahid Akhtar, Mysore, 570023 Karnataka (IN); Ravikumar, Bhadravathi, Mysore, 570 007 Karnataka (IN); Kulkarni, Ashok Krishna, Mysore, 570 023 Karnataka (IN)
(74) Representative: Coates, Ian Harold

(57) **Abstract**

A process is described for the preparation of (RR,SS)-2-dimethylaminomethyl-1-(3-methoxyphenyl) cyclohexanol hydrochloride (hereinafter 'tramadol hydrochloride'), which process comprises reacting an intermediate comprising one or more of:
(a) a mixture of (RR,SS)- and (RS,SR)-2-dimethylaminomethyl-1-(3-methoxyphenyl) cyclohexanol; and/or
(b) a hydrate of (RR,SS)-2-dimethylaminomethyl-1-(3-methoxyphenyl) cyclohexanol;
with HCl in the presence of a catalytic amount of water.

The process can be carried out in the absence of 1,4-dioxane or other category 1 carcinogenic solvent, and in one step, providing commercial advantages of simplicity, safety and purity. Tramadol hydrochloride is a known non-narcotic analgesic.

## Description

The present invention relates to a novel, one-pot process for the preparation of tramadol hydrochloride, which is (RR, SS)-2-dimethylaminomethyl-1-(3-methoxyphenyl)cyclohexanol hydrochloride (trans), from a mixture of its (RS, SR) (cis) and trans bases, and to an improved process for the preparation of tramadol (base) monohydrate, sometimes used as an intermediate in the preparation of tramadol hydrochloride.

Tramadol is a well-established drug disclosed in US patent specification no. 3 652 589, which is used in the form of its hydrochloride salt as a non-narcotic analgesic drug. Tramadol is the pharmacologically active trans isomer of 2-dimethylaminomethyl-1-(3-methoxyphenyl)cyclohexanol, as opposed to the corresponding cis isomer, namely, (RS, SR)-2-dimethylaminomethyl-1-(3-methoxyphenyl)cyclohexanol.

Various processes for the synthesis of tramadol hydrochloride have been described in the prior art. For example, US 3 652 589 and British patent specification no. 992 399 describe the preparation of tramadol hydrochloride. In this method, Grignard reaction of 2-dimethylaminomethyl cyclohexanone (Mannich base) with metabromo-anisole gives an oily mixture of tramadol and the corresponding cis isomer, along with Grignard impurities. This oily reaction mixture is subjected to high vacuum distillation at high temperature to give both the geometric isomers of the product base as an oil. This oil, on acidification with hydrogen chloride gas, furnishes insufficiently pure tramadol hydrochloride as a solid. This must then be purified, by using a halogenated solvent and 1,4-dioxane, to give sufficiently pure tramadol hydrochloride. The main drawback of this process is the use of large quantities of 1,4-dioxane and the need for multiple crystallizations to get sufficiently pure trans isomer hydrochloride (Scheme - 1).

The use of dioxane for the separation of tramadol hydrochloride from the corresponding cis isomer has many disadvantages, such as safety hazards by potentially forming explosive peroxides, and it is also a category 1 carcinogen (Kirk and Othmer, 3^{rd} edition, 17, 48). Toxicological studies of dioxane show side effects such as CNS depression, and necrosis of the liver and kidneys. Furthermore, the content of dioxane in the final tramadol hydrochloride has been strictly limited; for example, the German Drug Codex (Deutscher Arzneimittel Codex, DAC (1991)) restricts the level of dioxane in tramadol hydrochloride to 0.5 parts per million (ppm).

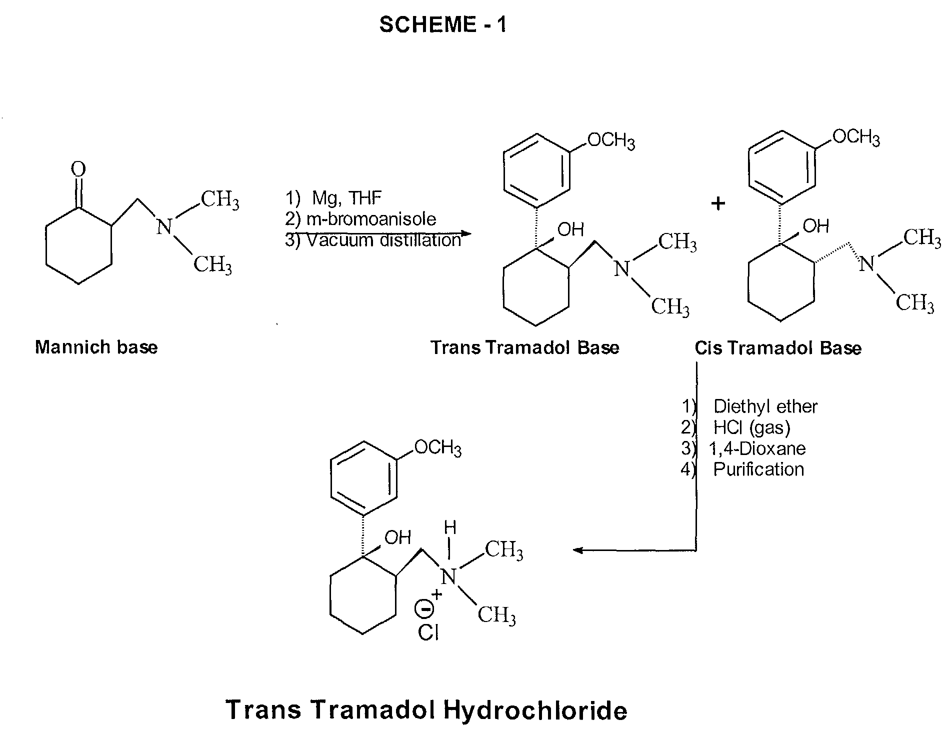

In another process, disclosed in US patent specification no. 5 414 129, the purification and separation of tramadol hydrochloride is undertaken from a reaction mixture containing the trans and cis isomers, and Grignard reaction side products, in which the reaction mixture is diluted in *iso*propyl alcohol and acidified with gaseous hydrogen chloride to yield (trans) tramadol hydrochloride (97.8%) and its cis isomer (2.2%), which is itself crystallized twice with isopropyl alcohol to give pure (trans) tramadol hydrochloride (Scheme - 2). This process relies on the use of multiple solvents to separate the isomers (*ie* butylacetate, 1-butanol, 1-pentanol, primary amyl alcohol mixture, 1-hexanol, cyclohexanol, 1-octanol, 2-ethylhexanol and anisole). The main drawback of this process is therefore in using high boiling solvents; furthermore, the yields of tramadol hydrochloride are still relatively low and the yield of the corresponding cis hydrochloride is relatively high in most cases.

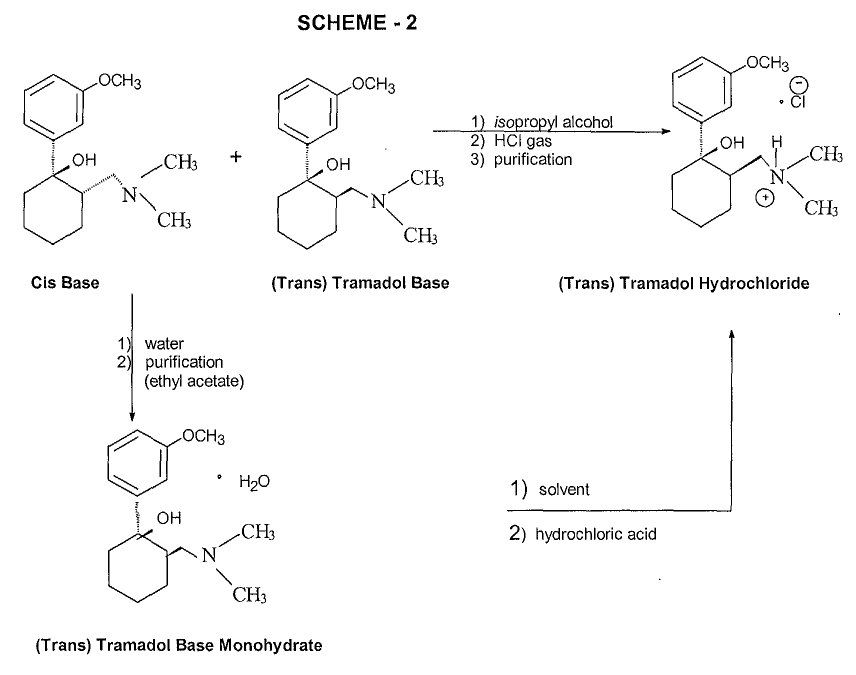

PCT patent specification no. WO 99/03820 describes a method of preparation of tramadol (base) monohydrate, which involves the reaction of Mannich base with metabromo-anisole (Grignard reaction) to furnish a mixture of tramadol base with its corresponding cis isomer and Grignard impurities. This, on treatment with an equimolar quantity of water and cooling to 0 to -5°C, gives a mixture of tramadol (base) monohydrate with the corresponding cis isomer (crude). It is further purified with ethyl acetate to furnish pure (trans) tramadol (base) monohydrate, which is again treated with hydrochloric acid in the presence of a suitable solvent to give its hydrochloride salt (Scheme - 2). The drawback of this method is that, to get pure (trans) tramadol hydrochloride, first is prepared pure (trans) tramadol (base) monohydrate, involving a two-step process, and this is then converted to its hydrochloride salt. The overall yield is low because of the multiple steps and tedious process involved.

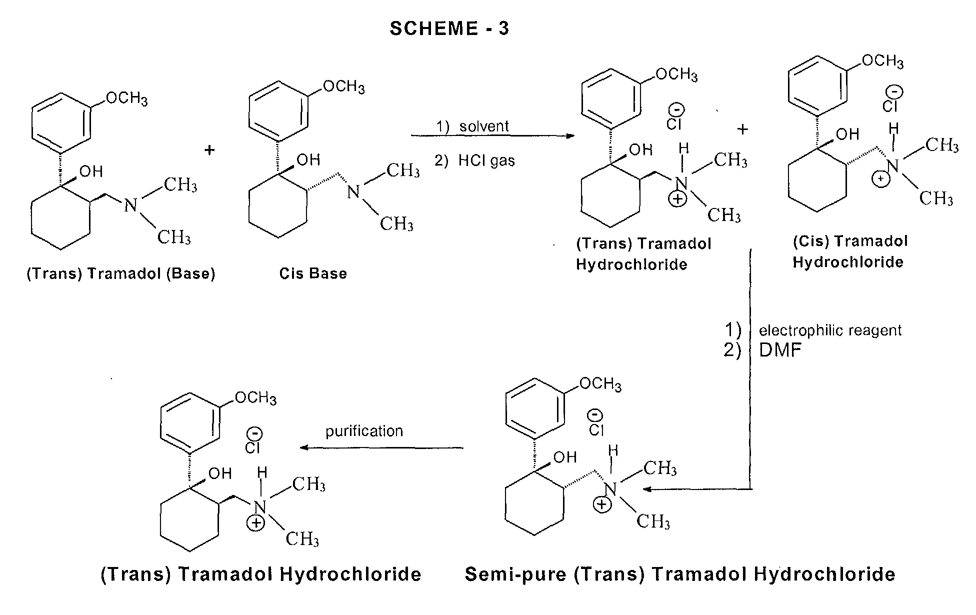

More recently, a process for the separation of tramadol hydrochloride from a mixture with its cis isomer, using an electrophilic reagent, has been described in US patent specification no. 5 874 620. The mixture of tramadol hydrochloride with the corresponding cis isomer is reacted with an electrophilic reagent, such as acetic anhydride, thionyl chloride or sodium azide, using an appropriate solvent (dimethylformamide or chlorobenzene) to furnish a mixture of tramadol hydrochloride (93.3 to 98.6%) with the corresponding cis isomer (1.4 to 6.66%), (Scheme - 3). The product thus obtained is further purified in *iso*propyl alcohol to give pure (trans) tramadol hydrochloride. However, the drawback of this process is that a mixture of tramadol base with its cis isomer is first converted into the hydrochloride salts, and this is further reacted with toxic, hazardous and expensive electrophilic reagents to get semi-pure (trans) tramadol hydrochloride. The content of the cis isomer is sufficiently high to require further purification, and this therefore results in a lower overall yield.

Therefore, all the known methods require potentially toxic solvents and/or reagents, and multiple steps to produce the desired quality and quantity of tramadol hydrochloride. By contrast, the present invention requires a single step process (or only two steps when tramadol hydrochloride is made *via* the tramadol (base) monohydrate route) using a natural solvent (*ie* water) in the absence of carcinogenic solvents (such as the category 1 carcinogen, 1,4-dioxane) to produce pure tramadol hydrochloride, so it is 'ecofriendly' and easily commercialized to plant scale without any difficulties.

We have surprisingly found that the required isomer ((RR, SS), *ie* tramadol hydrochloride) can be selectively isolated (*eg* by selective precipitation) by the action of water from a mixture of tramadol hydrochloride with the corresponding cis isomer. Therefore, tramadol hydrochloride can be prepared in a single step process from a cis + trans base mixture in the presence of HCI and water. Furthermore, the cis + trans base mixture may be converted to tramadol (base) monohydrate ((RR, SS), *ie* the trans isomer) under mildly basic conditions, and thereafter optionally converted to its hydrochloride salt in a similar manner.

Accordingly, the present invention provides a process for the preparation of (RR,SS)-2-dimethylaminomethyl-1-(3-methoxyphenyl) cyclohexanol hydrochloride (herein referred to as 'tramadol hydrochloride'), which process comprises reacting an intermediate comprising one or more of:
(a) a mixture of (RR,SS)- and (RS,SR)-2-dimethylaminomethyl-1-(3-methoxyphenyl) cyclohexanol; and/or
(b) a hydrate of (RR,SS)-2-dimethylaminomethyl-1-(3-methoxyphenyl) cyclohexanol;
with HCI in the presence of a catalytic amount of water.

The moisture content of the reaction mixture plays an important role in precipitating the tramadol hydrochloride, while the corresponding cis isomer remains in the mother liquor. The water content of the reaction mixture is preferably in the range of from 3.0 to 5.0% (based on the weight of the total reaction mixture). If the water content is less (0.5 to 3.0%), the unresolved cis isomer + tramadol HCI precipitates out. If the water content is greater than 5%, say 5-10%, then the yield decreases due to the high solubility of tramadol HCI in water. The water catalyst may be available from the use of concentrated hydrochloric acid (such as contains 65% water and 35% HCI) or from the use of hydrogen chloride gas with added water. Preferred is to use aqueous (concentrated) HCI, so that the reaction proceeds in one step.

Preferably, the reaction is carried out in the presence of a solvent, to assist in filtration of the precipitate and to make the reaction mixture homogeneous. More preferably, the reaction is carried out in the absence of 1,4-dioxane or other category 1 carcinogenic solvent. The solvent is suitably one in which tramadol hydrochloride is less soluble than is the corresponding cis isomer and which is water-miscible. Preferably, the solvent is low boiling, such as an aromatic or aliphatic hydrocarbon solvent, or mixture of solvents. Examples include: alcohols, such as ethyl alcohol, *iso*propyl alcohol, n-butanol and methoxyethanol; ketones, such as acetone and methylethylketone; acid nitriles, such as acetonitrile; cyclic ethers, such as tetrahydrofuran; and aromatic solvents, such as toluene; and mixtures thereof, such as toluene and *iso*propyl alcohol, and toluene and methylethylketone (see Table - 1 hereinbelow). The most suitable solvents are *iso*propyl alcohol and also methylethylketone, in which the purity and yield of trans tramadol are excellent.

The invention provides alternative ways of carrying out the process (Scheme - 4), for example by use of a mixture of tramadol (base) with the corresponding cis isomer plus water to form tramadol monohydrate and thereafter forming the HCI salt, as described further below. Preferred, however, is the one pot/step process described above.

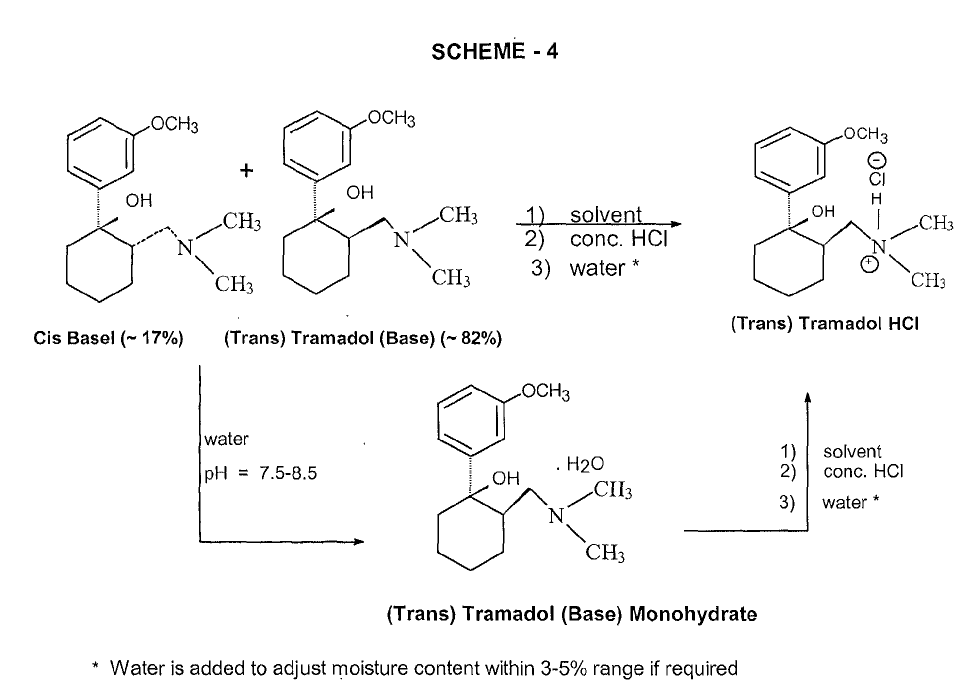

In another aspect (which demonstrates the role of water), the present invention provides a process for the preparation of (trans) tramadol hydrochloride, which comprises (a) reacting a mixture of tramadol (base) and the corresponding cis isomer with anhydrous hydrogen chloride gas in the presence of a solvent, such as one of those described above and preferably *iso*propyl alcohol, to furnish a mixture of tramadol hydrochloride and its cis isomer (its cis and trans ratio being similar to that of the starting material) and (b) adding a little water to the unresolved reaction mixture to achieve a water content of about 3-5%. From this two-step process, almost pure (trans) tramadol hydrochloride is prepared. This signifies that aqueous hydrochloric acid in the one step process acts both for the formation of the hydrochloride salt as well as providing the necessary water content of the reaction mixture to get the desired tramadol hydrochloride in a sufficiently pure form and good yield. This results in improved efficiency and is a novel method of preparation of tramadol hydrochloride from a mixture of the cis and trans bases.

Therefore, there is further provided a process according to the invention, wherein the concentrated hydrochloric acid is prepared *in situ* by combining a reaction mixture comprising the intermediate and anhydrous hydrogen chloride gas with water.

The invention further provides an improved method for preparing tramadol (base) monohydrate from a mixture of tramadol (base) with the corresponding cis isomer. The pH of the tramadol (base) mixture is adjusted to weakly alkaline (preferably, pH 7-10); if acidic, the yield decreases and if more basic (pH 10-13), separation of the trans monohydrate is more difficult. Therefore, there is still further provided process for the preparation of 2-dimethylaminomethyl-1-(3-methoxyphenyl) cyclohexanol monohydrate, which process comprises adjusting a mixture of (RR,SS)- and (RS,SR)-2-dimethylaminomethyl-1-(3-methoxyphenyl) cyclohexanol to a pH in the range of from about 7 to about 10.

The pH is more preferably in the range of from 7.5 to 8.5 and is suitably adjusted using a catalytic quantity of an acid, such as a mineral acid, aromatic acid or aliphatic organic acid.

When a mineral acid, such as sulphuric acid or hydrochloric acid, is used in the reaction to bring the pH to 7.5 to 8.5, the yield is lower in the case of hydrochloric acid because, at lower pH, it also forms some tramadol hydrochloride along with the tramadol (base) monohydrate. Tramadol hydrochloride is soluble in water, resulting in a lower yield (as mentioned in Table - 2), as compared to when sulphuric acid is used.

When an aromatic acid, such as benzoic acid or p-chlorobenzoic acid, is used to bring the pH to 7.5 to 8.5, the yield and quality of trans tramadol (base) monohydrate thus obtained is more or less the same.

Aliphatic organic acids, such as formic acid, acetic acid, monochloro-acetic acid, ethylhexanoic acid and phenylacetic acid, can also be used but, of these, acetic acid gives the best separation and improved yield of trans tramadol (base) monohydrate.

Conveniently, before adjusting the pH, the tramadol (base) mixture is diluted with water to facilitate filtration of the end-product. Preferably, about 8-10 w/v of water is present in the reaction mixture (*ie* about 8-10 grams of oily tramadol (base) mixture is taken in 80mL to 100mL of water). About 2% cis isomer is present in the tramadol (base) monohydrate thus prepared as a solid; it does not require further purification, *eg* in ethylacetate, contrary to the prior art method (PCT WO 99/03820).

Accordingly, in a preferred aspect, the present invention provides an improved method for the preparation of trans tramadol (base) monohydrate from a mixture of (RR, SS) and (RS, SR)-2-dimethylaminomethyl-1-(3-methoxyphenyl) cyclohexanol by treatment with water and acetic acid to pH 7.5 to 8.5 at 40-50°C to furnish (RR, SS)-2-dimethylaminomethyl-1-(3-methoxyphenyl) cyclohexanol (base) monohydrate having trans purity of more than 97%.

Formation of the monohydrate may be confirmed by IR spectrum and analysis of water content. The tramadol (base) monohydrate thus obtained may be converted into its hydrochloride salt, following the above-described method of this invention, in excellent purity and good yield.

Preferably, the reactions according to this invention are each carried out at elevated (*ie* above ambient) temperature, more preferably in the range of from 40-50°C, but less than 70°C.

The cis/trans mixture of (RR, SS) and (RS, SR)-2-dimethylaminomethyl-1-(3-methoxyphenyl) cyclohexanol is prepared by known procedures and may be used as a starting material in the following Examples.

The following Examples serve to illustrate the present invention. In each, the trans tramadol hydrochloride and its base monohydrate were determined by IR, ¹H-NMR, HPLC and melting point / mixed melting point with respect to a standard sample / authentic sample. Purity of cis / trans was generally determined by HPLC and found to be in the range of 97.0 to 99.8%. In the Tables, TMD represents 'tramadol'.

### Example 1 to 10 - Tramadol Hydrochloride : General Reaction Procedure

An oily mixture of cis and trans tramadol base (100g; trans 82.0%, cis = 17.0%) was acidified with concentrated hydrochloric acid (33g) at room temperature under stirring to pH 3 to 5. The water content of the reaction mixture was adjusted to 3-5%; if less were present, a catalytic amount of water was added into the reaction mixture to achieve the required moisture content (specified in Table - 1). The thick reaction mass was stirred at 40-50°C for 30 min. and water distilled out under vacuum to produce solid cis and trans tramadol hydrochloride. Solid cis and trans tramadol hydrochloride was dissolved in 200mL or 300mL of solvent (as specified in Table - 1) at 40-50°C. The reaction mixture was maintained at room temperature for 6 hours and then cooled at 0-5°C for 3 hours. The resulting white solid material was filtered and dried to constant weight at 50-55°C under vacuum to produce trans tramadol hydrochloride (specified in Table - 1 under "yield"). The purity was determined by HPLC as specified in Table - 1, below.

### Examples 11 to 19 - Tramadol (Base) Monohydrate : General Reaction Procedure

100gm of an isomeric mixture (as defined in Examples 1 to 10) of cis and trans tramadol base was diluted with 1000mL demineralised water at room temperature. The mixture was stirred for 5 min. and 1% diluted acid (as specified in Table - 2) added very slowly, dropwise, to pH 7.5 to 8.5. The reaction mixture was cooled and maintained for 3 hours at ambient temperature. The white solid was filtered and dried to constant weight at room temperature under vacuum to produce trans tramadol base monohydrate (as specified in Table - 2 below under "yield"). The purity of the trans tramadol base monohydrate was determined by HPLC as specified in Table - 2, below.

**Table - 1**

| **Ex.** **No.** | **Solvent** **used** | **Qty. of** **solvent** **used** **(mL)** | **Water** **content (%)** | **Yield(w/w)** | **Yield(w/w)** | **HPLC Analysis** | | |
|---|---|---|---|---|---|---|---|---|
| | | | | **Trans TMD HCl from TMD Base (Cis+Trans)** | **Trans TMD HCl from TMD Base (Trans)** | **Trans (%)** | **Cis (%)** | **Total other impurity** |
| 1 | Ethylalcohol | 200 | 4.20 | 0.14 | 0.17 | 99.73 | 0.20 | 0.07 |
| 2 | *Iso*propyl alcohol (IPA) | 200 | 3.15 | 0.58 | 0.70 | 99.47 | 0.41 | 0.12 |
| 3 | n-Butanol | 300 | 3.38 | 0.40 | 0.48 | 99.91 | 0.08 | 0.01 |
| 4 | Methoxyethanol | 200 | 3.00 | 0.20 | 0.24 | 99.00 | 0.90 | 0.10 |
| 5 | Acetone | 200 | 5.00 | 0.50 | 0.61 | 98.71 | 0.75 | 0.54 |
| 6 | Methylethylketone (MEK) | 200 | 3.12 | 0.52 | 0.63 | 99.26 | 0.18 | 0.56 |
| 7 | Acetonitrile | 200 | 3.20 | 0.45 | 0.54 | 98.20 | 1.34 | 0.46 |
| 8 | Tetrahydrofuran | 200 | 3.70 | 0.46 | 0.56 | 99.58 | 0.40 | 0.02 |
| 9 | Toluene: IPA | 300 | 3.12 | 0.44 | 0.53 | 99.63 | 0.22 | 0.15 |
| 10 | Toluene: MEK | 200 | 4.12 | 0.44 | 0.53 | 98.83 | 0.77 | 0.40 |

### Example 20 - Trans Tramadol Hydrochloride

The method was carried out according to that described in Examples 1-10, but replacing the mixture of cis and trans tramadol base with trans tramadol base monohydrate. This procedure, when using *iso*propyl alcohol solvent, as in Example 2, furnished 85.00gm of trans tramadol hydrochloride. Purity was determined as in Examples 1-10 and found to be: trans = 99.79%; Cis = 0.14%.

**Table - 2**

| **Ex.** **No.** | **Acid used** | **pH** | **Water** **content** **of TMD** **Base. H**_{**2**}**O** | **Yield w/w** | **Yield w/w** | **HPLC Analysis** | | |
|---|---|---|---|---|---|---|---|---|
| | | | | **Trans TMD Base. H**_{**2**}**O from TMD Base (Cis +Trans)** | **Trans TMD Base H**_{**2**}**O from TMD Base (Trans)** | **Trans (%)** | **Cis (%)** | **Total other impurity** |
| 11 | Formic | 8.24 | 6.25 | 0.65 | 0.79 | 98.84 | 0.60 | 0.56 |
| 12 | Hydrochloric | 8.00 | 6.30 | 0.55 | 0.67 | 98.66 | 1.10 | 0.24 |
| 13 | Sulphuric | 7.90 | 6.25 | 0.65 | 0.79 | 97.19 | 2.10 | 0.71 |
| 14 | Acetic | 8.30 | 6.25 | 0.71 | 0.86 | 97.22 | 2.04 | 0.74 |
| 15 | Monochloro-acetic | 8.26 | 6.15 | 0.67 | 0.81 | 97.63 | 1.87 | 0.50 |
| 16 | Ethylhexanoic | 8.35 | 6.20 | 0.62 | 0.75 | 97.01 | 2.19 | 0.80 |
| 17 | Benzoic | 8.00 | 6.35 | 0.65 | 0.79 | 98.00 | 1.46 | 0.54 |
| 18 | p-Chlorobenzoic | 8.30 | 6.30 | 0.63 | 0.76 | 97.20 | 2.10 | 0.70 |
| 19 | Phenylacetic | 8.40 | 6.21 | 0.64 | 0.78 | 98.5 | 1.18 | 0.32 |

## Claims

1. A process for the preparation of (RR,SS)-2-dimethylaminomethyl-1-(3-methoxyphenyl) cyclohexanol hydrochloride (hereinafter 'tramadol hydrochloride'), which process comprises reacting an intermediate comprising one or more of:
(a) a mixture of (RR,SS)- and (RS,SR)-2-dimethylaminomethyl-1-(3-methoxyphenyl) cyclohexanol; and/or
(b) a hydrate of (RR,SS)-2-dimethylaminomethyl-1-(3-methoxyphenyl) cyclohexanol;
with HCl in the presence of a catalytic amount of water.

2. A process according to claim 1, carried out in the absence of 1,4-dioxane or other category 1 carcinogenic solvent.

3. A process according to claim 1 or claim 2, wherein the water content of the reaction mixture is preferably in the range of from 3.0 to 5.0% (based on the weight of the total reaction mixture).

4. A process according to any preceding claim, wherein the HCI is in the form of concentrated hydrochloric acid.

5. A process according to claim 4, wherein the concentrated hydrochloric acid is prepared *in situ* by combining a reaction mixture comprising the intermediate and anhydrous hydrogen chloride gas with water.

6. A process according to any preceding claim, wherein the reaction is carried out in the presence of a solvent.

7. A process according to claim 6, wherein the solvent is one in which the tramadol hydrochloride is less soluble than the corresponding cis isomer and which is water-miscible.

8. A process according to any preceding claim, wherein the reaction is carried out in *iso*propyl alcohol and/or methylethylketone.

9. A process according to any preceding claim, wherein the intermediate comprises a mixture of (RR,SS)- and (RS,SR)-2-dimethylaminomethyl-1-(3-methoxyphenyl) cyclohexanol.

10. A process according to any of claims 1 to 8, wherein the intermediate comprises 2-dimethylaminomethyl-1-(3-methoxyphenyl) cyclohexanol monohydrate.

11. A process according to claim 10, wherein the 2-dimethylaminomethyl-1-(3-methoxyphenyl) cyclohexanol monohydrate is itself prepared from a mixture of (RR,SS)- and (RS,SR)-2-dimethylaminomethyl-1-(3-methoxyphenyl) cyclohexanol at a pH in the range of from about 7 to about 10.

12. A process for the preparation of 2-dimethylaminomethyl-1-(3-methoxyphenyl) cyclohexanol monohydrate, which process comprises adjusting a mixture of (RR,SS)-and (RS,SR)-2-dimethylaminomethyl-1-(3-methoxyphenyl) cyclohexanol to a pH in the range of from about 7 to about 10.

13. A process according to claim 11 or claim 12, wherein the preparation of the monohydrate is carried out in the presence of acetic acid.

14. A process according to any of claims 11 to 13, wherein the mixture of (RR,SS)-and (RS,SR)-2-dimethylaminomethyl-1-(3-methoxyphenyl) cyclohexanol) is diluted with water.

15. A process according to any of claims 1 to 9 or 12, which process is carried out in one step.
